# DEMANDE DE BREVET EUROPEEN

(11) **EP 0 737 444 A1**
(43) Date de publication de la demande: **16.10.1996**
(21) Numéro de dépôt: 96400784.3
(22) Date de dépôt: 11.04.1996
(51) Int. Cl.: A61B 17/15

(54) **Guide de coupe rotulienne**

(30) Priorité: 13.04.1995 FR 9504465
(71) Demandeur: FRANCE-BLOC, F-95270 Viarmes (FR)
(72) Inventeur: Wen, Ning, 60500 Chantilly (FR); Charpenet, Rémy, 88000 Epinal (FR); Coudane, Henri, Hôpital Central, 54035 Nancy (FR); Roure, Jean-Luc, 94370 Sucy en Brie (FR); Dongar, Christian, 95270 Viarmes (FR); Junger, Jean-Claude, Lab. d'Anatomie, Bât.AB, 54500 Vandoeuvre les Nancy (FR)
(74) Mandataire: Rinuy, Santarelli

(57) **Abrégé**

Guide permettant de réaliser une coupe rotulienne précise pour la mise en place de la rotule prothétique de la prothèse de genou utilisant un composant fémoral d'essai, comportant:
i)- une platine de glissement (20) d'un guide de broche (30),
ii)- un guide de broche (30) comportant au moins deux canaux (33) de guidage de broche (50) installés perpendiculairement au plan sagittal du condyle, et comportant deux panneaux présentant deux surfaces (32, 34) disposées à angle droit, le premier panneau présentant une surface plane (32) et une lumière centrale (31), le second panneau présentant vers l'intérieur du guide une surface plane (34),
iii) un élément de serrage (40) coopérant avec la tige (23) pour bloquer le guide de broche (30) par rapport à la platine de glissement (20) et éviter ainsi les mouvements de translation.

## Description

La présente invention concerne un guide permettant de réaliser une coupe rotulienne précise pour la mise en place de la rotule prothétique de la prothèse de genou.

L'invention trouve son domaine d'application dans la mise en place de la rotule prothétique d'une prothèse tri-compartimentale de genou. Nous rappelons que la prothèse est constituée de trois composants :
- le composant fémoral
- le composant tibial
- le composant rotulien

La pose de la rotule prothétique est souvent la dernière étape dans la mise en place d'une prothèse de genou.

Pour obtenir celle-ci, il est nécessaire d'enlever une épaisseur d'os de la rotule anatomique englobant la surface articulaire. Elle sera remplacée par une rotule prothétique ayant la même épaisseur que celle de l'os enlevé.

Le bon fonctionnement d'une prothèse de genou ne peut être obtenu qu'en respectant l'isométrie et la tension des ligaments ; cela ne peut être fait qu'en réalisant des coupes précises en épaisseur et en orientation pour l'implantation des éléments prothétiques.

Pour réaliser la coupe rotulienne, il est nécessaire de retourner la rotule anatomique de façon à visualiser la surface articulaire. Celle-ci sera maintenue à l'aide d'une pince dont les mâchoires serviront de plan de coupe afin de réaliser la coupe rotulienne. Le placement de la pince se fait visuellement en essayant d'avoir une répartition uniforme de l'os. La coupe ainsi réalisée peut être inclinée et donner un mauvais placement de l'implant rotulien qui aura tendance à se luxer.

L'épaisseur de coupe rotulienne est actuellement déterminée en prenant comme référence la crête de la rotule anatomique. La rotule anatomique étant souvent usée, cette crête anatomique l'est aussi et ne peut donc être une bonne référence pour la coupe. Les coupes ainsi réalisées sont parfois trop minces, ce qui entraîne une rotule prothétique surélevée et une limitation de la flexion, ou trop épaisses ce qui fragilise la rotule anatomique et peut entraîner des fractures.

Il existe aussi une autre méthode qui définit le plan de coupe de la rotule anatomique par rapport à la trochlée du composant fémoral. Cette méthode nécessite que le genou soit fléchi à un angle fixe, flexion à laquelle la rotule anatomique se trouve engagée dans la gorge du composant fémoral d'essai. On suppose que la rotule anatomique est parfaitement stable dans la trochlée prothétique. En prenant une référence sur le composant fémoral, on peut effectuer la coupe rotulienne qui permettra de mettre le composant rotulien sans perturber la cinématique du genou.

Malheureusement la trochlée du composant fémoral ne présente pas une forme complémentaire comme la trochlée anatomique ; elle est beaucoup moins creuse, ce qui permet à la rotule anatomique de basculer dans la trochlée prothétique et entraîne une erreur de placement (orientation et épaisseur) du composant rotulien.

L'objectif de la présente invention est de proposer un guide de coupe rotulienne pour la pose du composant rotulien dans la mise en place d'une prothèse tri-compartimentale de l'articulation du genou. Ce guide permet de réaliser une coupe rotulienne permettant d'avoir une bonne épaisseur de coupe ainsi qu'une bonne orientation du plan de coupe.

Pour réaliser une coupe rotulienne correcte, il est nécessaire d'avoir un repère stable et précis. Ceci entraîne bien évidemment que la rotule anatomique doit être stable.

Le guide de coupe rotulienne selon la présente invention se monte sur le composant fémoral d'essai en mettant par exemple deux ergots du guide de coupe dans deux trous cylindriques complémentaires réalisés sur les côtés du composant fémoral d'essai. On obtient ainsi le placement du guide de coupe par rapport au composant fémoral d'essai.

Le guide de coupe selon l'invention se décompose en une platine de glissement définissant un plan rigoureusement perpendiculaire aux coupes osseuses du condyle fémoral (coupes réalisées pour la mise en place du composant fémoral), un guide de broche qui peut à la fois effectuer une translation et pivoter sur la platine ci-dessus afin d'être placé à la fois dans le plan de la rotule anatomique, schématisé dans une vue de profil, par une ligne liée entre l'insertion du tendon quadricipital et celle du ligament rotulien et être en contact avec la surface articulaire (forme de profil) du composant fémoral d'essai, et un élément de serrage tel qu'un écrou coopérant avec un filetage permettant de bloquer la position relative entre les différentes pièces citées ci-dessus. On définit ainsi l'orientation et l'épaisseur de la coupe en prenant comme référence la surface articulaire du composant fémoral d'essai. Le guide de broche dispose de préférence de plus de 2 canaux de guidage, avantageusement cylindriques, qui permettent de planter dans la rotule anatomique des clous qui servent de support de guidage pour réaliser la coupe rotulienne.

C'est pourquoi la présente invention a pour objet un guide permettant de réaliser une coupe rotulienne précise pour la mise en place de la rotule prothétique de la prothèse de genou utilisant un composant fémoral d'essai, caractérisé en ce qu'il comporte :
i)- une platine de glissement d'un guide de broche, ladite platine comportant du côté opposé au composant fémoral d'essai une surface de glissement plane, parallèle au plan sagittal du condyle du composant fémoral d'essai, et une tige installée perpendiculairement à cette surface de glissement et comportant du côté du composant fémoral d'essai au moins une protubérance de forme complémentaire d'un évidement prévu dans le composant fémoral d'essai pour se fixer à celui-ci, platine sur laquelle vient glisser
ii)- un guide de broche comportant au moins deux canaux de guidage de broche installés perpendiculairement au plan sagittal du condyle, et comportant deux panneaux présentant deux surfaces disposées à angle droit,
   le premier panneau, parallèle au plan sagittal, présentant
   - une surface plane parallèle au plan sagittal, pouvant glisser en contact avec la surface de glissement de la platine
   - une lumière centrale permettant le passage de la tige au travers du premier panneau, de dimensions suffisantes pour autoriser la translation du guide de broche dans toutes les directions parallèlement au plan sagittal
      le second panneau, perpendiculaire au premier panneau, situé du côté du composant fémoral d'essai, présentant vers l'intérieur du guide une surface plane de dimension suffisante pour venir en contact avec la surface articulaire d'une prothèse fémorale d'essai, pendant les mouvements de translation du guide de broche, et comportant en outre les canaux de guidage de broche,
iii) un élément de serrage coopérant avec la tige pour bloquer le guide de broche par rapport à la platine de glissement et éviter ainsi les mouvements de translation.

Les éléments essentiels de la platine de glissement sont sa surface de glissement plane, la tige limitant les déplacements de la platine de glissement et utilisée pour le blocage du guide de broche, et la protubérance venant coopérer avec le composant fémoral d'essai pour se fixer à celui-ci.

La tige installée perpendiculairement à la surface de glissement est avantageusement une tige filetée ; elle constitue ainsi un moyen simple, pouvant coopérer avec un écrou de filetage correspondant, pour bloquer le guide de broche par rapport à la platine de glissement et ainsi éviter les mouvements de translation de ce dernier. Tout autre moyen permettant de serrer le guide de broche contre la platine de glissement, tel qu'un système à levier ou un système à excentrique est bien évidement utilisable.

La protubérance située sur la platine de glissement et permettant sa fixation sur le composant fémoral d'essai est par exemple un élément parallélépipédique. Il peut s'agir également d'une tige de section hexagonale ou carrée par exemple. Dans des conditions préférentielles de réalisation, et comme illustré sur les figures, il s'agit de deux tétons ou doigts pouvant venir s'insérer à frottement doux dans deux orifices latéraux prévus par exemple sur chacun des condyles du composant fémoral d'essai.

La fonction essentielle du guide de broche, consiste à pouvoir glisser parallèlement au plan sagittal du condyle du composant fémoral d'essai grâce à une surface plane parallèle à ce plan sagittal.

Cette surface plane située sur un premier panneau comprend une lumière permettant le passage de la tige au travers du premier panneau.

Afin de permettre la translation du guide de broche dans toutes les directions parallèlement au plan sagittal, cette lumière est avantageusement allongée dans la direction correspondant à un déplacement de cette pièce selon la surface articulaire fémorale. Sa largeur sera avantageusement au moins une fois et demie et de préférence environ le double de la largeur de la tige située sur la platine de glissement. Cette lumière, au niveau de la surface de glissement, aura par exemple, comme illustré sur les dessins, une forme rectangulaire, de préférence aux angles arrondis.

Comme il apparaît à l'homme de l'art, la forme de la surface du premier panneau opposé à la surface plane pouvant glisser en contact avec la surface de glissement de la platine, n'est pas critique. Cependant, cette surface est avantageusement plane et parallèle à ladite surface pouvant glisser en contact avec la surface de glissement de la platine. Ainsi, on peut obtenir un blocage facile du guide de broche en utilisant une vis située sur la platine de glissement, et un écrou de serrage et de blocage.

L'élément essentiel du second panneau du guide de broche, est sa surface plane, de dimension suffisante pour venir en contact avec la surface articulaire d'une prothèse fémorale d'essai. De ce fait, la surface de ce panneau opposée à la surface venant en contact avec la surface articulaire peut avoir une forme quelconque. Pour des raisons pratiques de fabrication, cette surface sera avantageusement parallèle à la surface précitée.

Ainsi dans des conditions préférentielles de réalisation, le guide de broche a grossièrement la forme de deux parallélépipèdes rectangles aplatis installés perpendiculairement l'un à l'autre et de section transversale générale en forme de L. Ce second panneau est en outre traversé par les canaux de guidage de broche. Ces canaux sont donc installés perpendiculairement à la surface de glissement de la platine de glissement et perpendiculairement au premier panneau du guide de broche. Ces canaux sont au minimum au nombre de deux, et sont avantageusement au nombre de trois ou quatre, préférentiellement au nombre de trois.

La description qui va suivre en regard des dessins annexés, donnés à titre d'exemple illustratif, fera bien comprendre en quoi consiste l'invention et comment elle peut être réalisée et utilisée.

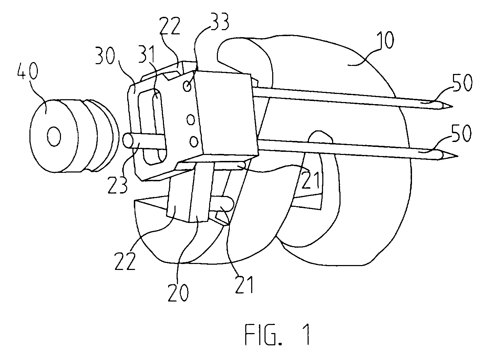

La figure 1 est une vue en perspective du guide de coupe rotulien monté sur le composant fémoral d'essai.

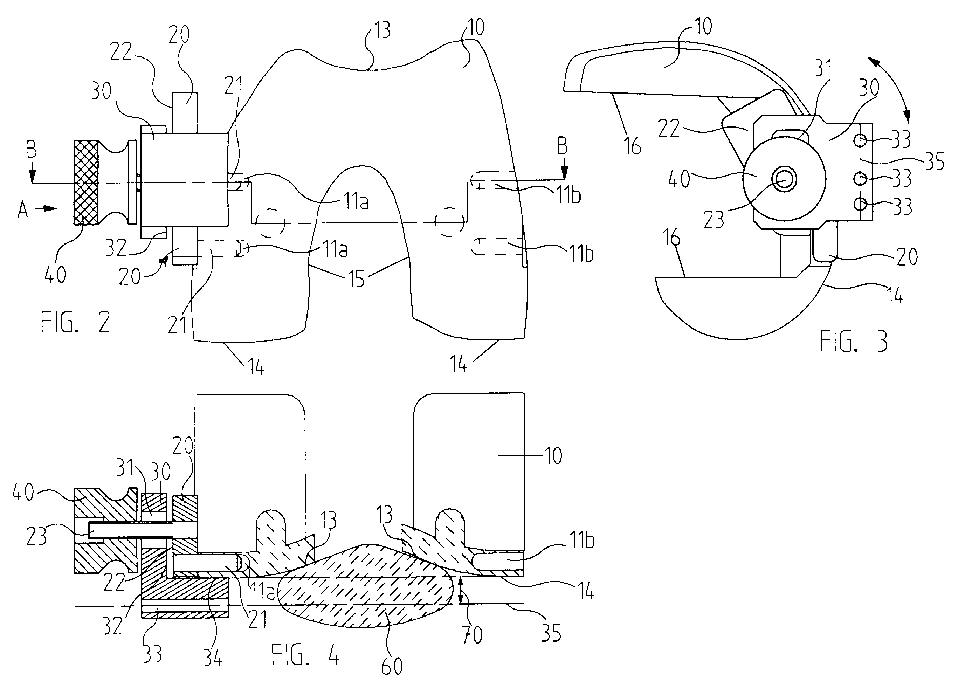

La figure 2 est une vue de face du même guide.

La figure 3 est une vue de côté suivant A en se référant à la vue de face de la figure 2.

La figure 4 représente la coupe suivant B-B' de la pièce en vue de face.

En référence à ces dessins, le dispositif comporte, d'une part un composant fémoral d'essai 10 du type ayant la même forme que le composant fémoral définitif, et d'autre part, un ancillaire guide de coupe rotulien comprenant quatre parties, une platine de glissement 20 fixée sur le composant fémoral d'essai 10, un guide de broches 30 glissé sur la platine 20, un écrou de serrage 40 permettant de bloquer la position du guide de broches 30 par rapport à la platine 20, et des broches 50 de diamètre standard prévues pour être implantées dans la rotule anatomique 60 guidées par les trois canaux tubulaires du guide de broches 30 et servir de la référence de coupe rotulienne.

Le composant fémoral d'essai 10 du type décrit est symboliquement composé d'une gorge de trochlée 13 descendant de la partie supéro-antérieure jusqu'à la partie antéro-distale (à cet endroit la gorge de la trochlée 13 est coupée par l'échancrure intercondylienne 15), sur laquelle la rotule anatomique 60 ou la rotule prothétique se glisse, d'une surface articulaire 14 aplatie, des plans de coupe osseuse 16 et de deux trous cylindriques 11a ou 11b, dont l'axe est parallèle au plan de coupe osseuse 16 et perpendiculaire au plan de profil du composant fémoral d'essai, à la fois sur le bord interne et le bord externe. En effet, pour installer une prothèse totale de genou, lors de l'intervention, on accède à l'articulation du genou du côté interne de préférence ; on utilise alors les deux trous 11 situés sur le bord interne du condyle du composant fémoral d'essai utilisé. L'accès à l'articulation du genou par la voie externe est rare.

Quand le genou est fortement fléchi, la rotule anatomique 60 se trouve au dessus de l'échancrure 15 et sa crête ne touche plus le fond de la trochlée 13.

La platine de glissement 20 est composée essentiellement de deux protubérances en forme de tétons 21 cylindriques s'insérant dans les deux trous cylindriques latéraux du condyle fémoral d'essai 11a ou 11b de taille correspondante, d'une surface de glissement 22 perpendiculaire aux trous latéraux 11a, 11b ou aux plans de coupe osseuse 16 du composant fémoral d'essai et d'une tige filetée 23.

Le guide de broche 30 est une pièce en forme, en coupe, de "L" à angle droit, présentant une surface de glissement 32 glissant sur la surface de glissement 22 de la platine. Il est constitué de deux panneaux sensiblement parallélépipédiques perpendiculaires entre eux, à section transversale en "L". Le premier panneau glissant sur la platine 20 comporte une lumière 31 dont la largeur est supérieure au diamètre de la tige filetée 23, permettant à la fois une bonne mobilité du guide de broches 30 par rapport à la platine de glissement 20 tout en limitant le déplacement. Le second panneau comprend plusieurs canaux 33 tubulaires pour guider les broches 50 dont l'axe 35 de broches 50 ou de canaux tubulaires 33 est perpendiculaire au plan de profil du composant fémoral d'essai. Le second panneau comprend en outre une surface plane de contact 34 destinée à être mise en contact tangentiel avec la surface articulaire 14 du composant fémoral d'essai. Avec une telle disposition, on obtient une épaisseur de coupe connue 70 par rapport au composant fémoral d'essai.

L'écrou de serrage 40 permet, après un placement correct du guide de broche 30 de bloquer celui-ci avant de mettre en place des broches 50 dans la rotule anatomique 60.

La distance 70 entre la surface articulaire 14 aplatie et l'axe 35 de broche 50 étant connue, on peut ainsi régler l'épaisseur de coupe rotulienne par rapport à la surface articulaire 14 du composant fémoral d'essai 10.

Le guide selon l'invention est destiné à réaliser une coupe rotulienne précise en épaisseur et en orientation et trouve de ce fait sa place dans les ancillaires de prothèse de genou.

## Revendications

1. Guide permettant de réaliser une coupe rotulienne précise pour la mise en place de la rotule prothétique de la prothèse de genou utilisant un composant fémoral d'essai, caractérisé en ce qu'il comporte :
i)- une platine de glissement (20) d'un guide de broche (30), ladite platine comportant du côté opposé au composant fémoral d'essai une surface (22) de glissement plane, parallèle au plan sagittal du condyle du composant fémoral d'essai, et une tige (23) installée perpendiculairement à cette surface (22) de glissement et comportant du côté du composant fémoral d'essai au moins une protubérance (21) de forme complémentaire d'un évidement (11a) prévu dans le composant fémoral d'essai pour se fixer à celui-ci, platine sur laquelle vient glisser
ii)- un guide de broche (30) comportant au moins deux canaux (33) de guidage de broche (50) installés perpendiculairement au plan sagittal du condyle, et comportant deux panneaux présentant deux surfaces (32, 34) disposées à angle droit,
le premier panneau, parallèle au plan sagittal, présentant
- une surface plane (32) parallèle au plan sagittal, pouvant glisser en contact avec la surface (22) de glissement de la platine (20),
- une lumière centrale (31) permettant le passage de la tige (23) au travers du premier panneau, de dimensions suffisantes pour autoriser la translation du guide de broche (30) dans toutes les directions parallèlement au plan sagittal
le second panneau, perpendiculaire au premier panneau, situé du côté du composant fémoral d'essai, présentant vers l'intérieur du guide une surface plane (34) de dimension suffisante pour venir en contact avec la surface articulaire (14) d'une prothèse fémorale d'essai, pendant les mouvements de translation du guide de broche (30), et comportant en outre les canaux (33) de guidage de broche (50),
iii) un élément de serrage (40) coopérant avec la tige (23) pour bloquer le guide de broche (30) par rapport à la platine de glissement (20) et éviter ainsi les mouvements de translation.

2. Guide selon la revendication 1, caractérisé en ce que la tige (23) est filetée, et l'élément de serrage (40) est du type écrou.

3. Guide selon la revendication 1 ou 2, caractérisé en ce que la platine de glissement (20) comporte deux protubérances (21) en forme de tétons allongés.

4. Ensemble de coupe rotulien comprenant un guide tel que défini à l'une des revendications 1 à 3, ainsi qu'un composant fémoral d'essai comprenant un évidement (11a, 11b) de forme complémentaire prévu dans au moins un des condyles du composant fémoral d'essai.
